# DEMANDE DE BREVET EUROPEEN

(11) **EP 0 841 042 A1**
(43) Date de publication de la demande: **13.05.1998**
(21) Numéro de dépôt: 97402662.7
(22) Date de dépôt: 06.11.1997
(51) Int. Cl.: A61F 2/46, A61F 2/34

(54) **Ensemble d'ancillaires pour la pose de cotyles de prothèses de hanche, et ensemble prothétique de cotyle prêt à être posé**

(30) Priorité: 12.11.1996 FR 9613761
(71) Demandeur: PPC, 55000 Bar le Duc (FR)
(72) Inventeur: Charpenet, Rémy, 88000 Epinal (FR); Paulin, Marc, 52000 Chaumont (FR); Preaut, Jacques, 55000 Bar Le Duc (FR)
(74) Mandataire: Martin, Jean-Paul

(57) **Abrégé**

Ensemble d'ancillaires pour la pose de cotyles (1) de prothèses de hanche du type à corolle sensiblement hémisphérique, constituée de pétales expansibles (2) pourvus de picots extérieurs (5), caractérisé en ce qu'il comprend un obturateur (7) de fermeture des pétales (2) sous contrainte radiale élastique dans une position de rapprochement les uns des autres, des moyens (8, 9) de maintien de cet obturateur (7) sur les bords des pétales (2) par liaison avec le fond du cotyle (1), un ancillaire d'impaction (11) permettant d'impacter le cotyle (1) en libérant ses pétales (2) par extraction de l'obturateur (7); l'ensemble comporte également une platine (12) d'expansion des pétales (2) pouvant être introduite dans ceux-ci après extraction de l'obturateur (7) et libération des pétales (2), et un ancillaire (13) permettant d'enfoncer la platine (12) dans le cotyle (1) pour provoquer l'expansion radiale des pétales (2) après l'impaction, afin d'assurer l'ancrage du cotyle (1) par pénétration des picots d'ancrage (5) dans la paroi osseuse cotyloïdienne. Cet agencement facilite considérablement la mise en place par le chirurgien du cotyle (1) par impaction des pétales (2) dans la cavité cotyloïdienne, puis expansion radiale des pétales (2) au moyen de la platine (12) et de l'ancillaire (13) d'expansion. Cotyle (1) muni du dispositif ancillaire tel que précédemment décrit.

## Description

La présente invention a pour objet un ensemble d'ancillaires pour la pose de cotyles artificiels de prothèses de hanche, du type à corolle sensiblement hémisphérique, constituée de pétales expansibles pourvus de picots extérieurs d'ancrage osseux. L'invention a également pour objet un dispositif ancillaire solidarisable et incorporable auxdits cotyles pour permettre la présentation et leur mise en place dans le cotyle naturel du patient.

On connaît des cotyles du type à corolle, formés d'un ensemble de pétales expansibles dont les bases se rejoignent au niveau de la calotte polaire du cotyle. Ces pétales présentent une certaine élasticité qui permet de les rapprocher sensiblement les uns des autres dans la direction radiale. Sur leur surface extérieure, ces pétales sont munis de picots assurant l'encrage dans la paroi osseuse cotyloïdienne lorsque le chirurgien provoque, au moyen d'un outillage approprié, une expansion radiale des pétales.

Cependant l'impaction et l'ancrage osseux de ces cotyles à corolle présentent certaines difficultés, que l'invention vise à surmonter au moyen d'un ensemble approprié d'ancillaires.

Conformément à l'invention, l'ensemble d'ancillaires pour la pose de cotyles de prothèses de hanche du type à corolle constituée de pétales expansibles pourvus de picots extérieurs, comprend un couvercle-obturateur de fermeture des pétales sous contrainte radiale élastique dans une position de rapprochement les uns des autres, des moyens de maintien de ce couvercle-obturateur sur les bords des pétales par liaison avec le fond du cotyle, un ancillaire d'impaction permettant d'impacter le cotyle en libérant ces pétales par l'extraction du couvercle-obturateur, une platine d'expansion des pétales pouvant être introduite dans ceux-ci après extraction du couvercle-obturateur et libération des pétales, et un ancillaire pourvu de moyens d'enfoncement de la platine dans le cotyle pour permettre l'expansion radiale des pétales après l'impaction, afin d'assurer l'ancrage du cotyle par pénétration des picots d'ancrage dans la paroi osseuse cotyloïdienne.

Conformément à l'invention, le cotyle du type à corolle précité est réalisé, assemblé et livré par le fabricant en position de pose, c'est-à-dire comportant le couvercle-obturateur en position de maintien des pétales sous contrainte radiale élastique de telle façon que son diamètre équatorial extérieur total soit, lors de sa présentation dans le cotyle naturel du patient, inférieur à celui du cotyle naturel et que les pétales, après avoir été libérés, puissent conférer au cotyle artificiel le diamètre équatorial souhaité, supérieur au diamètre équatorial en position de pétales contraints.

Suivant d'autres caractéristiques de l'invention :
- les moyens de maintien et de liaison du couvercle-obturateur avec une zone polaire formant le fond du cotyle comprennent une douille taraudée pourvue d'un doigt terminal fileté pouvant se visser axialement dans un trou taraudé correspondant de la zone polaire, et un bouchon comportant une tête et une extrémité filetée pouvant traverser axialement le couvercle-obturateur de manière à venir se visser dans la douille tandis que sa tête s'appuie sur la face extérieure du couvercle-obturateur; le filetage de l'extrémité du bouchon est vissé à gauche tandis que l'ancillaire d'impaction et d'extraction du couvercle-obturateur présente un doigt terminal ayant un filetage vissé à droite dans un trou taraudé complémentaire du bouchon ; de ce fait le vissage à droite du doigt de l'ancillaire dans le bouchon provoque le dévissage de celui-ci et son extraction de la douille, ce qui permet l'extraction du couvercle-obturateur.

En variante le filetage du doigt peut être réalisé pour permettre un vissage à gauche tandis que le filetage de l'extrémité du bouchon est alors réalisé pour permettre un vissage à droite.
- L'ancillaire d'impaction et d'extraction du couvercle-obturateur comporte un axe monté libre en translation à l'intérieur d'une poignée tubulaire, une extrémité de l'axe est munie dudit doigt fileté adapté pour se visser dans le bouchon tandis que son extrémité opposée porte une poignée de manoeuvre; la poignée tubulaire comporte à l'une de ses extrémités au moins un ergot adapté pour venir s'introduire dans un trou complémentaire du couvercle obturateur, afin d'empêcher la rotation de ce dernier pendant le dévissage du bouchon de retenue par l'axe.
- L'ancillaire.d'expansion radiale des pétales du cotyle comprend une tige axiale munie d'une extrémité adaptée pour pouvoir être fixée à la douille elle-même solidaire de la zone polaire du cotyle, une poignée tubulaire traversée axialement par la tige et pouvant être vissée sur une partie filetée de cette dernière, et un manchon monté libre en rotation et en translation sur la tige entre la poignée tubulaire et l'extrémité coopérant avec la douille, de telle sorte que le vissage de la poignée tubulaire exerce sur le manchon une poussée axiale transmise à la platine qui s'enfonce dans les pétales et provoque leur expansion radiale.

Un tel ensemble d'ancillaires facilite considérablement la tâche du chirurgien, en lui permettant dans un premier stade, au moyen du premier ancillaire d'impaction, de libérer les pétales de la pression élastique de retenue qu'ils subissent du fait du couvercle-obturateur, de telle sorte qu'ils prennent leur position libre détendue dans la paroi cotyloïdienne. Dans une seconde étape, le chirurgien utilise le second ancillaire et la platine associée introduite dans le cotyle en position sensiblement équatoriale, afin d'enfoncer cette platine dans le cotyle et ainsi de provoquer l'écartement radial des pétales et leur ancrage par les picots dans l'os sous chondral.

D'autres particularités et avantages de l'invention apparaîtront au cours de la description qui va suivre, faite en référence aux dessins annexés qui en illustrent deux formes de réalisation à titre d'exemples non limitatifs.

La figure 1 est une vue en perspective éclatée d'une forme de réalisation du cotyle de prothèse de hanche, du couvercle-obturateur et des moyens de liaison de ce dernier avec le fond du cotyle, ce dernier étant représenté mis en place dans une cavité cotyloïdienne de hanche.

La figure 2 est une vue en élévation latérale et coupe partielle correspondant à la figure 1 et à échelle légèrement agrandie.

La figure 3 est une vue en plan de l'intérieur du cotyle suivant la flèche K de la figure 2.

La figure 4 est une vue en élévation latérale du cotyle des figures 1 à 3 muni du couvercle-obturateur de fermeture.

La figure 5 est une vue en élévation suivant la flèche L de la figure 4.

La figure 6 est une vue en élévation longitudinale d'une forme de réalisation de l'ancillaire d'impaction du cotyle des figures 1 à 5.

La figure 7 est une vue en perspective du cotyle équipé d'une platine d'expansion radiale de ses pétales et d'une partie de l'ancillaire correspondant.

La figure 8 est une vue en élévation longitudinale de l'ancillaire d'expansion radiale du cotyle partiellement représenté à la figure 7.

La figure 9 est une vue en élévation partielle d'une seconde forme de réalisation de l'ancillaire d'expansion radiale de la figure 8 et en coupe longitudinale de la douille associée adaptée pour être fixée dans le fond du cotyle.

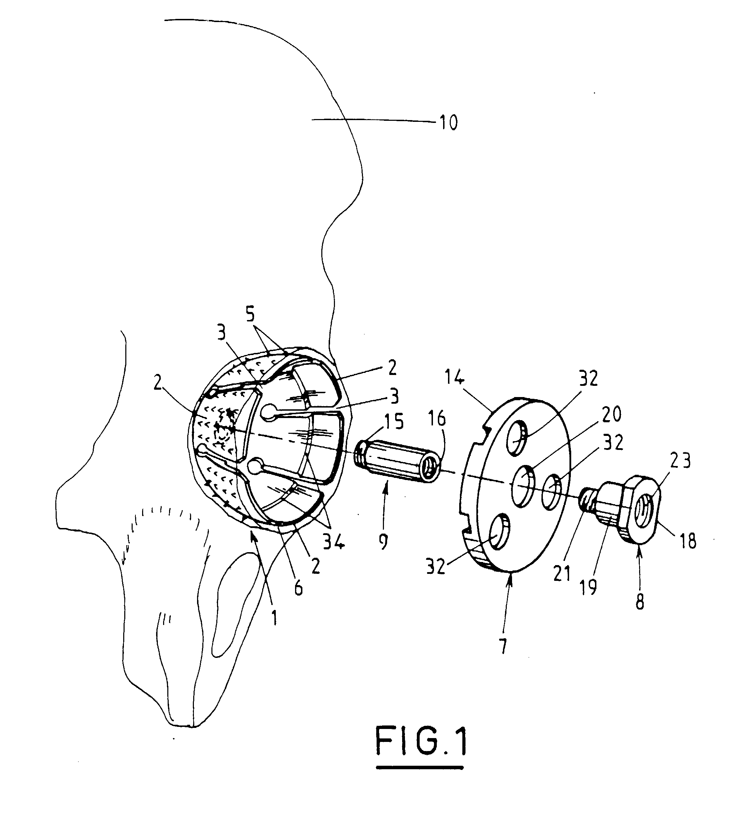

On a représenté aux dessins un cotyle 1 sensiblement hémisphérique, du type à corolle constituée d'une série de pétales expansibles 2, séparées par des fentes 3. Les pétales 2 se rejoignent à leur base au niveau de la calotte polaire 4 du cotyle 1, et sont munis sur leur surface extérieure d'un ensemble de picots 5 formant des pointes d'ancrage osseux dans la paroi de la cavité cotyloïdienne 6 de l'os de bassin 10 (figure 1).

Le cotyle 1 peut être impacté dans la cavité cotyloïdienne 6 et ses pétales ancrés par les picots ou pointes 5, après expansion radiale, au moyen d'un ensemble d'ancillaires décrits ci-dessous.

Cet ensemble comprend un couvercle-obturateur 7 constituant un couvercle de fermeture des pétales 2, des organes 8, 9 de maintien du couvercle-obturateur 7 sur les bords équatoriaux des pétales 2 et de liaison avec le fond 4 du cotyle 1, un ancillaire 11 d'impaction permettant d'impacter le cotyle dans la cavité 6; l'ensemble d'ancillaires comporte également une platine 12 pouvant être introduite dans le cotyle 1 après extraction du couvercle-obturateur 7 et un second ancillaire 13 pouvant coopérer avec la platine 12 pour provoquer l'expansion radiale des pétales 2 et corrélativement leur ancrage dans la paroi cotyloïdienne par les pointes ou picots 5.

On décrira tout d'abord le couvercle-obturateur 7 et ses organes de maintien 8, 9.

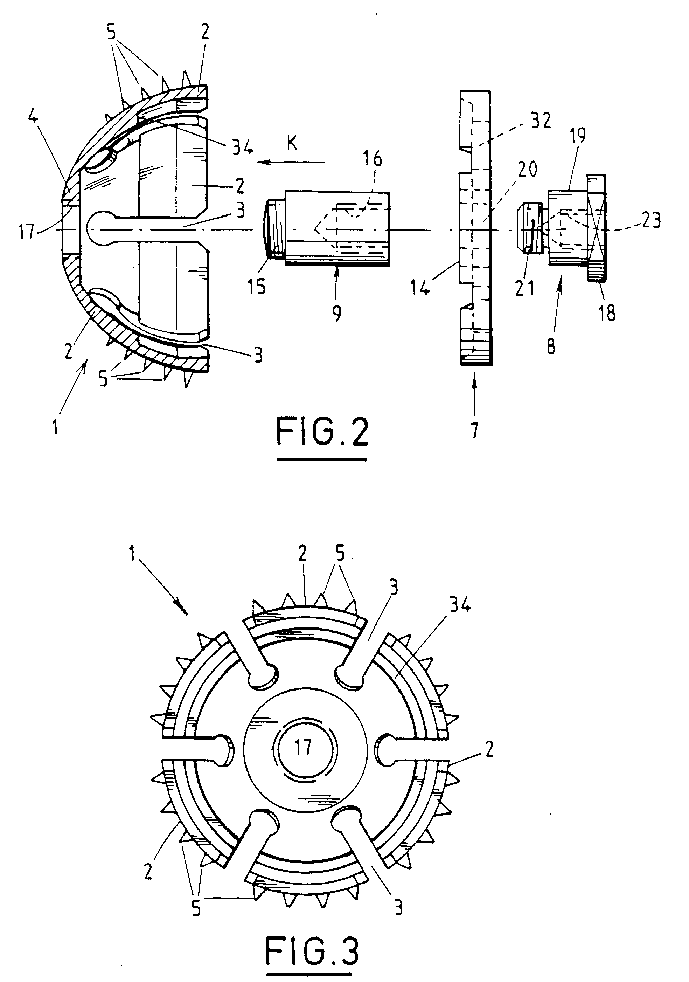

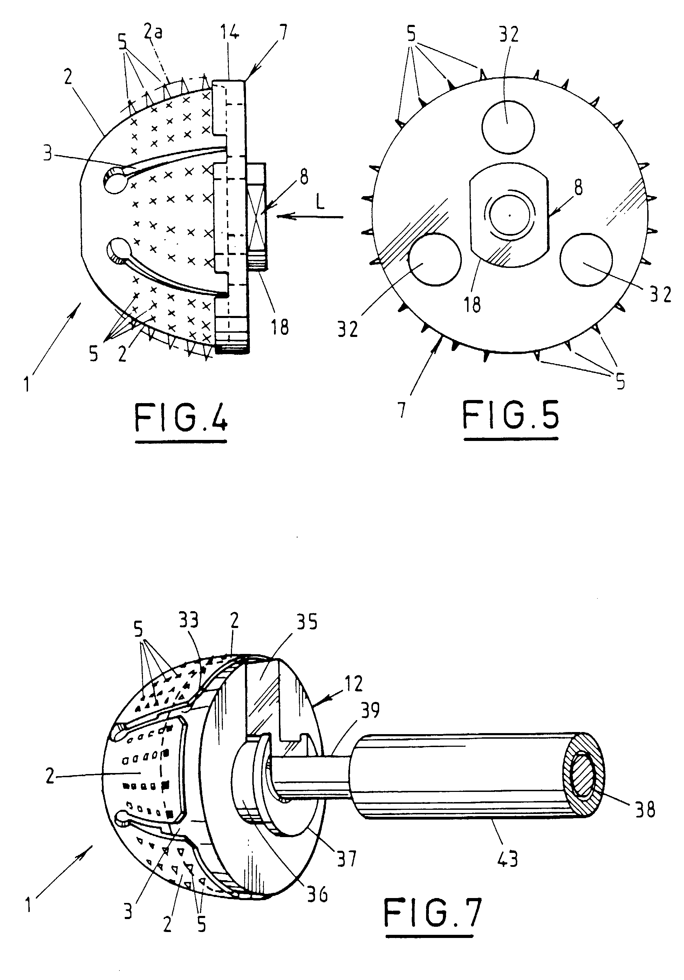

Les pétales 2 présentent une relative élasticité qui leur permet d'être comprimés radialement lorsqu'ils sont coiffés par le couvercle-obturateur 7, lequel présente à cet effet une collerette périphérique 14 (figures 1, 2 et 4) de retenue des pétales 2 en position comprimée rapprochée radialement. Pour pouvoir remplir cette fonction, le couvercle circulaire 7 et sa collerette 14 ont un diamètre légèrement inférieur à celui des pétales 2 à l'état libre détendu (figures 1 et 2) : cette différence de diamètre apparaît nettement à la figure 4 où l'on a représenté partiellement les pétales 2a en position libre détendue, et en trait continu ces mêmes pétales comprimés et rapprochés par introduction de leurs extrémités à l'intérieur de la collerette 14.

Les moyens de maintien et de liaison du couvercle-obturateur 7 avec la zone polaire 4 formant le fond du cotyle 1 comprennent une douille taraudée 9 pourvue d'un doigt terminal fileté 15 et d'un trou borgne taraudé 16. Le doigt 15 peut venir se visser axialement dans un trou taraudé 17 correspondant ménagé axialement dans la zone polaire 4; Le dispositif de maintien du couvercle-obturateur 7 sur le cotyle 1 comporte également un bouchon 8 présentant une tête 18, un corps 19 et une extrémité filetée 21. Le corps 19 et l'extrémité 21 peuvent traverser axialement le couvercle-obturateur 7 par un trou central 20 de celui-ci, afin de permettre le vissage de l'extrémité 21 dans le trou borgne 16 après vissage du doigt 15 dans le trou 17 et mise en place du couvercle obturateur 7 grâce à un rapprochement, par des moyens connus en soi, des pétales 2.

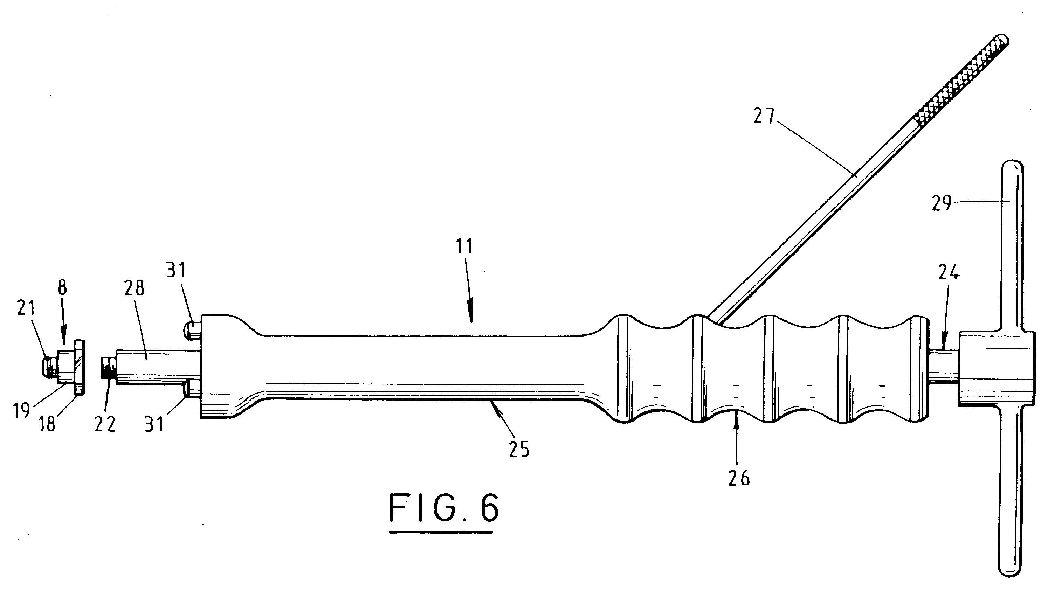

Le filetage de l'extrémité 21 du bouchon 8 est avantageusement vissé à gauche, tandis que l'ancillaire 11 d'impaction présente un doigt terminal 22 (figure 6) ayant un filetage vissé à droite dans un trou taraudé complémentaire 23 du bouchon 8. Le filetage de l'extrémité 15 de la douille 9 est vissé à droite. De ce fait, le vissage à droite du doigt 22 de l'ancillaire 11 d'impaction (qui sera décrit en détail ci-après) dans le trou taraudé 23 provoque le dévissage du bouchon 8 par rapport à la douille 9 et son extraction de celle-ci, ce qui permet la libération et l'extraction du couvercle-obturateur 7.

En variante le filetage de l'extrémité 22 de l'ancillaire 11 pourrait être fileté à gauche et inversement le filetage de l'extrémité 21 du bouchon 8 être fileté à droite.

Suivant une variante d'exécution non représentée, la douille 9 est du type à baïonnette et adaptée pour coopérer avec un outil dont une extrémité est munie d'un ergot correspondant, l'introduction de ce dernier dans le dispositif à baïonnette de la douille permettant de dévisser et d'enlever la douille.

L'ancillaire 11 d'impaction et d'extraction du couvercle-obturateur 7 comporte (figure 6) un axe 24 monté libre en translation à l'intérieur d'une poignée tubulaire 25 présentant avantageusement une zone cannelée 26 facilitant sa préhension manuelle par le chirurgien, ainsi qu'un levier latéral 27 de manutention. Une extrémité 28 de l'axe 24 est munie du doigt fileté 22 adapté pour se visser dans le bouchon 8, tandis que son extrémité opposée porte une poignée 29 de manoeuvre. La poignée tubulaire 25 comporte à l'une de ses extrémités au moins un ergot 31, par exemple trois ergots régulièrement répartis angulairement et dont deux sont visibles à la figure 6. Ces ergots 31 sont adaptés pour venir s'introduire dans des trous complémentaires 32 du couvercle-obturateur 7 (figure 1), afin d'empêcher la rotation de ce dernier pendant le dévissage du bouchon 8 de retenue par l'axe 24.

Pour procéder à l'impaction du cotyle 1, le chirurgien procède de la manière suivante.

Initialement l'assemblage tel que représenté aux figures 4 et 5, et constitué par le cotyle 1, le couvercle-obturateur 7, le bouchon 8 et la douille 9 est fabriqué, monté et livré ainsi au chirurgien qui peut disposer du reste de l'ancillaire.

Le cotyle 1 est coiffé par le couvercle-obturateur 7 maintenu en place sur les pétales 2 en les comprimant radialement par le bouchon 8 et la douille 9. Cette dernière est vissée par son extrémité 15 dans le trou taraudé 17 et le bouchon 8 est vissé à travers le trou 20 par son doigt fileté 21 dans le trou taraudé 16 de la douille 9. Le chirurgien introduit l'extrémité filetée 22 dans le trou taraudé 23 en vissant à droite, tout en faisant pénétrer les ergots 31 dans les trous 32. De ce fait le couvercle-obturateur 7 est empêché de tourner tandis que le chirurgien poursuit, à l'aide de la poignée de manoeuvre 29, le vissage de l'extrémité 22 de l'axe 24 dans le bouchon 8. Ce dernier ayant été vissé à gauche, la poursuite du vissage à droite de l'axe 24 dévisse le bouchon 8 de la douille 9. Son extraction de cette dernière et du couvercle-obturateur 7 libère ce dernier, de sorte que par détente élastique radiale, les pétales 2 viennent prendre leur position d'impaction 2a (figure 4) dans laquelle les picots 5 pénètrent partiellement dans la paroi osseuse.

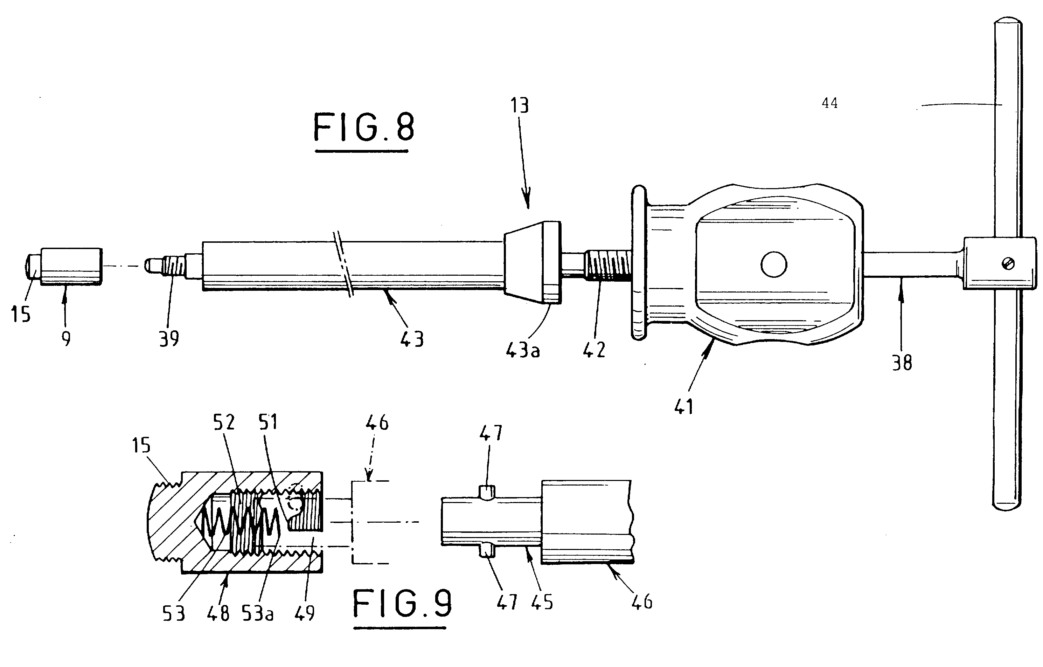

L'expansion radiale des pétales 2 pour permettre leur ancrage définitif dans la paroi de la cavité cotiloïdienne 6 est effectuée par le chirurgien au moyen du dispositif d'ancillaire représenté aux figures 7 à 9.

Ce dispositif comporte la platine 12 qui présente une surface tronconique 33 et est dimensionnée de manière à pouvoir être introduite axialement dans le cotyle 1, en exerçant une pression croissante sur les pétales 2 afin de provoquer leur expansion radiale. Cette expansion prend fin lorsque la platine 12 vient en appui de butée sur un épaulement 34 formé annulairement sur la paroi intérieure des pétales 2 (figures 1, 2, 3). La platine 12 présente en outre une fente radiale 35 et est pourvue d'un collet central 36 terminé par une collerette circulaire 37 interrompue, de même que le collet 36, par la fente 35. Cet agencement permet de manipuler la platine 12 par une pince à saisir (non représentée), connue en soi et appliquée sur le collet 36.

L'ancillaire 13 d'expansion radiale des pétales 2 comprend (figure 8) une tige axiale 38 munie d'une extrémité 39 adaptée pour pouvoir être fixée à la douille 9 elle-même solidaire de la zone polaire 4 du cotyle 1. L'ancillaire 13 comprend également une poignée tubulaire 41, réalisée avantageusement de manière ergonomique, traversée axialement par la tige 38 et pouvant être vissée sur cette dernière, qui comporte à cet effet une zone filetée 42 en prise avec un alésage taraudé correspondant de la poignée tubulaire 41. L'ancillaire 13 comporte également un manchon 43 monté libre en rotation et en translation sur la tige 38 entre la poignée tubulaire 41 et l'extrémité 39. Cette dernière peut être filetée (figure 8) et adaptée pour se visser dans la douille 9. L'extrémité opposée de la tige 38 est munie d'une poignée transversale 44 permettant sa manoeuvre en rotation.

La mise en oeuvre de la platine 12 et de l'ancillaire d'expansion 13 s'effectue de la manière suivante.

La platine 12 étant introduite à l'intérieur des pétales 2 (figure 7) et la douille 9 étant vissée dans le trou 17 de la calotte polaire 4, le chirurgien introduit l'extrémité filetée 39 dans la fente 35 pour la visser dans la douille 9. Puis il fait coulisser le manchon 43 jusqu'à ce qu'il vienne en butée contre la collerette 37. Enfin le chirurgien visse la poignée ergonomique 41 sur la partie filetée 42 jusqu'à ce que cette poignée 41 vienne en appui sur l'extrémité 43a du manchon 43, et exerce sur celui-ci une poussée axiale transmise par le manchon 43 à la platine 12. Il en résulte que celle-ci s'enfonce dans les pétales 2 et provoque leur expansion radiale, ainsi que l'ancrage des picots 5 dans la paroi osseuse de la cavité 6. La poignée 41 est mise en rotation dans le sens horaire, jusqu'à pénétration complète de la platine tronconique 12 dans le cotyle 1 et mise en butée sur l'épaulement d'arrêt 34. L'expansion puissante des pétales 2 permet l'ancrage des picots 5 dans l'os sous chondral.

Une fois l'ancrage effectué, le chirurgien dévisse la poignée ergonomique 41 dans le sens anti-horaire, puis retire la platine 12 à l'aide d'une pince à saisir (non représentée). La douille 9 est également dévissée.

Suivant une seconde forme de réalisation (figure 9), l'extrémité 45 de la tige axiale 46 comporte au moins un ergot transversal 47, de préférence deux, et la douille 48 présente un agencement à baïonnette à deux fentes longitudinales 49 et deux crans correspondants 51 de retenue des ergots 47. Ces éléments à baïonnette sont formés dans la paroi d'un trou taraudé 52, sur le fond duquel peut prendre appui un ressort intérieur 53, dont une extrémité 53a coopère avec la partie terminale 45 pour maintenir les ergots 47 dans les crans 51 du système à baïonnette.

Ainsi le trou taraudé 52 peut recevoir, soit une tige axiale 38 à extrémité filetée 39, soit une tige axiale 46 à extrémité 45 munie des ergots 47.

## Revendications

1. Ensemble d'ancillaires pour la pose de cotyles (1) de prothèses de hanche du type à corolle sensiblement hémisphérique, constituée de pétales expansibles (2) pourvus de picots extérieurs (5), caractérisé en ce qu'il comprend un couvercle-obturateur (7) de fermeture des pétales sous contrainte radiale élastique dans une position de rapprochement les uns des autres, des moyens (8, 9) de maintien de ce couvercle-obturateur sur les bords des pétales par liaison avec le fond (4) du cotyle, un ancillaire d'impaction (11) permettant d'impacter le cotyle en libérant ses pétales par extraction du couvercle-obturateur, une platine (12) d'expansion des pétales pouvant être introduite dans ceux-ci après extraction de l'obturateur et libération des pétales, et un ancillaire (13) pourvu de moyens d'enfoncement de la platine dans le cotyle pour permettre l'expansion radiale des pétales après l'impaction, afin d'assurer l'ancrage du cotyle par pénétration des picots d'ancrage dans la paroi osseuse cotyloïdienne.

2. Ensemble selon la revendication 1, caractérisé en ce que le couvercle obturateur (7) comporte une collerette périphérique (14) de retenue des pétales (2) dont le diamètre est légèrement inférieur à celui des pétales à l'état libre (2a).

3. Ensemble selon la revendication 2, caractérisé en ce que lesdits moyens de maintien et de liaison du couvercle-obturateur (7) avec une zone polaire formant le fond (4) du cotyle (1) comprennent une douille taraudée (9) pourvue d'un doigt terminal fileté (15) pouvant se visser axialement dans un trou taraudé (17) correspondant de la zone polaire, et un bouchon (8) comportant une tête (18) et une extrémité filetée (21) pouvant traverser axialement le couvercle-obturateur (7), de manière à venir se visser dans la douille (9) tandis que sa tête s'applique sur la face extérieure du couvercle-obturateur, en ce que le filetage de l'extrémité (21) du bouchon est vissé à gauche tandis que l'ancillaire (11) d'impaction et d'extraction du couvercle-obturateur présente un doigt terminal (22) ayant un filetage vissé à droite dans un trou taraudé (23) complémentaire du bouchon (8), de telle sorte que le vissage à droite du doigt de l'ancillaire dans le bouchon provoque le dévissage de celui-ci, et son extraction de la douille, ce qui permet l'extraction du couvercle-obturateur, ou inversement le filetage du doigt est vissé à gauche et le filetage de l'extrémité du bouchon est vissé à droite.

4. Ensemble selon la revendication 3, caractérisé en ce que la douille (9) est du type à baïonnette et adaptée pour coopérer avec un outil ayant une extrémité munie d'un ergot correspondant, dont l'introduction dans le dispositif à baïonnette permet de dévisser et d'enlever la douille.

5. Ensemble selon la revendication 4, caractérisé en ce que l'ancillaire d'impaction (11) et d'extraction du couvercle-obturateur (7) comporte un axe (24) monté libre en translation à l'intérieur d'une poignée tubulaire (25), en ce qu'une extrémité (28) de l'axe est munie dudit doigt fileté (22) adapté pour se visser dans le bouchon (8), tandis que son extrémité opposée porte une poignée (29) de manoeuvre, en ce que la poignée tubulaire (25) comporte à l'une de ses extrémités au moins un ergot (31) adapté pour venir s'introduire dans un trou complémentaire (32) du couvercle-obturateur (7), afin d'empêcher la rotation de ce dernier pendant le dévissage du bouchon de retenue par l'axe.

6. Ensemble selon l'une quelconque des revendications 1 à 5, caractérisé en ce que la platine d'expansion (12) est tronconique et peut être enfoncée dans la corolle du cotyle (1) jusqu'à un épaulement (34) de butée formé annulairement sur la paroi intérieure des pétales (2).

7. Ensemble selon la revendication 6, caractérisé en ce que la platine (12) présente une fente radiale (35) et un collet central (36) de préhension par une pince à saisir, terminé par une collerette (37) de retenue.

8. Ensemble selon la revendication 6 ou 7, caractérisé en ce que l'ancillaire d'expansion radiale (13) des pétales (2) du cotyle (1) comprend une tige axiale (38) munie d'une extrémité (39) adaptée pour pouvoir être fixée à la douille (9) elle-même solidaire de la zone polaire (4) du cotyle (1), une poignée tubulaire (41) traversée axialement par la tige et pouvant être vissée sur une partie filetée (42) de cette dernière, et un manchon (43) monté libre en rotation et en translation sur la tige entre la poignée tubulaire et l'extrémité coopérant avec la douille, de telle sorte que le vissage de la poignée tubulaire exerce sur le manchon une poussée axiale transmise à la platine qui s'enfonce dans les pétales et provoque leur expansion radiale.

9. Ensemble selon la revendication 8, caractérisé en ce que l'extrémité (39) de la tige axiale (38) est filetée et adaptée pour se visser dans la douille (9).

10. Ensemble selon la revendication 8, caractérisé en ce que l'extrémité de la tige axiale (46) comporte au moins un ergot transversal (47) et la douille (48) présente un agencement à baïonnette (49, 51) adapté pour recevoir l'ergot, ainsi qu'un ressort intérieur (53) prenant appui sur le fond d'un trou taraudé (51) de la douille pour maintenir l'extrémité (45) de la tige dans l'agencement à baïonnette, ledit trou taraudé pouvant ainsi recevoir soit une tige axiale (38) à extrémité filetée (39), soit une tige axiale (46) à extrémité (45) munie d'au moins un ergot (47).

11. Ensemble prothétique de cotyle (1) adapté à des éléments d'ancillaire selon l'une quelconque des revendications précédentes, dans lequel le cotyle, qui est préparé pour être prêt à être prosé, est du type à corolle sensiblement hémisphérique, constitué de pétales expansibles (2), caractérisé en ce qu'il comprend un couvercle-obturateur (7) adapté pour fermer et pour maintenir les pétales (2) dans une position de rapprochement les uns des autres, et des moyens (8, 9) de maintien de ce couvercle-obturateur sur les bords des pétales par liaison avec le fond (4) du cotyle.

12. Ensemble selon la revendication 11, caractérisé en ce que lesdits moyens de maintien comprennent une douille (9) taraudée, pouvant se visser par son extrémité (15) dans la zone polaire du cotyle (1), et un bouchon (8) extérieur au couvercle-obturateur (7), traversant celui-ci pour se visser dans la douille (9) et maintenir le couvercle-obturateur sur les pétales.
